# EUROPEAN PATENT SPECIFICATION

(11) **EP 4 476 532 B1**
(45) Date of publication and mention of the grant of the patent: **28.01.2026**
(21) Application number: 23704139.7
(22) Date of filing: 10.02.2023
(51) Int. Cl.: G01N 21/84

(54) **METHODS AND DEVICES FOR DETERMINING THE CONCENTRATION OF AT LEAST ONE ANALYTE IN A BODILY FLUID**
VERFAHREN UND VORRICHTUNGEN ZUR BESTIMMUNG DER KONZENTRATION VON MINDESTENS EINEM ANALYTEN IN EINER KÖRPERFLÜSSIGKEIT
PROCÉDÉS ET DISPOSITIFS DE DÉTERMINATION DE LA CONCENTRATION D'AU MOINS UN ANALYTE DANS UN FLUIDE CORPOREL

(30) Priority: 11.02.2022 EP 22156307
(43) Date of publication of application: 18.12.2024
(73) Proprietor: F. Hoffmann-La Roche AG, 4070 Basel (CH); Roche Diabetes Care GmbH, 68305 Mannheim (DE)
(72) Inventor: BERG, Max, 68305 Mannheim (DE); HAILER, Fredrik, 68305 Mannheim (DE); LIMBURG, Bernhard, 68305 Mannheim (DE)
(74) Representative: Riwotzki, Karsten
(86) International application number: PCT/EP2023/053265
(87) International publication number: WO 2023/152266

(56) References cited:
- WO-A1-2020/251460
- US-A1- 2011 076 695
- US-A1- 2017 073 728
- US-A1- 2019 302 009
- US-A1- 2020 386 672

## Description

### Technical Field

The present invention refers to a determination method of determining a color expectation range for assessing the plausibility of a color formation value obtained in an analytical measurement based on a color formation reaction. The invention further relates to a measurement method of performing an analytical measurement. Further, the invention relates to a determination system and a mobile device as well as to computer programs, computer-readable storage media and a kit. The methods, systems, mobile devices, the computer programs, the computer-readable storage media and the kit may be used specifically in medical diagnostics, in order to for example quantitatively or qualitatively detect one or more analytes in one or more body fluids and/or bodily fluids, such as for detecting glucose in blood and/or interstitial fluid. Other fields of application of the present invention, however, are feasible.

### Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the con-centration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test elements.

Generally, devices and methods known to the skilled person make use of test elements and/or test strips comprising one or more test chemicals, which, in presence of the analyte to be detected, are capable of performing one or more detectable detection reactions, such as optically detectable detection reactions. With regard to the test chemicals comprised in test elements and/or test strips, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

In analytical measurements, specifically analytical measurements based on color formation reactions, one technical challenge resides in the evaluation of the color change which is due to the detection reaction. Besides using dedicated analytical devices, such as handheld blood glucose meters, the use of generally available electronics such as smart phones and portable computers or other mobile devices has become more and more popular over the recent years. Thus, a camera comprised by these mobile devices may be used to measure the color change of the detection reaction in the test elements and/or test strips. As opposed to laboratory measurements and measurements performed by using dedicated pairings of analytical measurement devices and test elements and/or test strips, when using mobile computing devices such as smart phones, various influences need to be taken into account. As an example, lighting conditions, positioning, vibrations, electronic defects or other more or less uncontrollable influences are to be considered. Generally, procedures may be used which comprise mathematically correcting and/or compensating consequences of such influences on the color change by making use of color reference for photometric measurements.

US 11,112,406 B2 describes a method for providing immunoassay test results including collecting at least one biologic with a testing device. The method further includes conjugating the biologic with particles on a conjugate pad of a test strip to create an immune complex, binding antigens or antibodies of the immune complex to antigens or antibodies of a test line, providing a software application to be stored on a mobile device having a camera; capturing an image of the testing device, including a color mosaic having at least one color value corresponding to a positive test result, comparing the color values of the test line image to the color values of the image of the color mosaic, determining if the color values of the image of the test line are within a predetermined range of the at least one color value of the image of the color mosaic corresponding to a positive test result; and presenting test results on the viewing screen.

CN 104969068 B describes a method and device for performing color-based reaction testing of biological materials. The method includes capturing and interpreting digital images of an unexposed and later exposed instrument within an automatically calibrated environment. The instrument includes a Unique Identification (UID) label, Reference Color Bar (RCB) providing samples of standardized colors for image color calibration, and several test-specific sequences of Chemical Test Pads (CTP). The method further includes locating the instrument in the image, extracting the UID, extracting the RCB, and locating the plurality of CTP in each image. The method further reduces image noise in the CTP and calibrates the image automatically according to lighting measurements performed on the RCB. The method further determines test results by comparing the color of the CTP image to colors in a Manufacturer Interpretation Color Chart (MICC). The method shows these results in graphical or quantified mode.

EP 2 916 117 A1 describes color quantification of chemical test pads and titration of analytes that can be performed under different lighting conditions. In one embodiment, the lighting condition is estimated under which a digital image is captured and utilized to select a set of reference colors from which the quantified color is compared to determine the titration. In another embodiment, a plurality of comparisons is made with different lighting conditions with the result having the highest confidence level being selected to determine the titration.

US 2015/0247874 A1 describes a method of detecting a presence of an analyte in a sample. The method includes selecting an aptamer selective to the analyte and binding the aptamer to a nanoparticle. The nanoparticles having a free state are perceived as a first color and an aggregate state are perceived as a second color. The sample is introduced to the nanoparticle-bound aptamers, and aggregation of the nanoparticles is promoted. A colorimetric change is analyzed, wherein the aggregate state of the nanoparticles is achieved in the presence of the analyte in the sample.

US 2011/076695 A1 describes an immunoassay analyzer capable of discriminating between normal coloring due to a specific immunoreaction and abnormal coloring due to a cause other than the specific immunoreaction in a measurement region of a sample analysis tool. The immunoassay analyzer includes an optical detection unit and a determination unit. The optical detection unit includes an optical signal measurement unit for measuring an optical signal at each of two or more different wavelengths including a main wavelength for detecting color change due to the specific immunoreaction and a sub-wavelength(s) other than the main wavelength. The determination unit includes a discrimination unit for comparing the respective optical signals at the two or more different wavelengths and discriminating between the color change due to the specific immunoreaction and color change due to a cause other than the specific immunoreaction based on a comparison criterion determined previously.

US 2020/386672 A1 describes a calibration method for calibrating a camera for detecting an analyte in a sample. A plurality of different color coordinate systems and a set of test samples are provided. The test samples are applied to test elements that have test fields for producing an optically detectable reaction. Images of the colored test fields are acquired using the camera and color coordinates for the images are generated. The color coordinates that are generated are transformed into a set of measured concentrations by using a set of coding functions. The set of measured concentrations is compared with the known concentrations of the test samples and a best match color coordinate system of the plurality of color coordinate systems is determined. A best match coding function of the plurality of coding functions is also determined.

US 2017/073728 A1 describes analytical apparatuses for detecting at least one analyte in a sample, where in an analyte measurement at least an electrical or optical property changeable by presence of the analyte at least one test chemical of a test element is recorded, and where the analytical apparatus also can per-form at least one quality measurement on the at least one test chemical such as an intrinsic luminescence, which is recorded and from the intrinsic luminescence a conclusion is drawn on a quality of the test chemical and thus the test element. Methods also are disclosed for detecting at least one analyte in a sample that include a quality measurement of the at least one test chemical of the test strip.

Despite the advantages achieved by the known methods and devices, several technical challenges remain. Specifically, user-dependent influences, such as wrongful handling and/or inappropriate storage of test elements and/or test strips or other more or less uncontrollable user-dependent handling errors may lead to undetected color changes. Such undetected color changes may result in an inaccurate analytical measurement when determining the analyte concentration based on a color formation reaction.

### Problem to be solved

It is therefore desirable to provide methods and devices that at least partially address the above-mentioned technical challenges. Specifically, it is desirable to provide methods and devices which allow for a detection of color changes introduced by wrongful and/or inappropriate user handling.

### Summary

This problem is addressed by a determination method of determining a color expectation range for assessing the plausibility of a color formation value obtained in an analytical measurement based on a color formation reaction according to claim 1, and by a measurement method of performing an analytical measurement according to claim 8. Further, by a determination system according to claim 11, a mobile device according to claim 14, and a kit with the features of claim 15 Advantageous embodiments are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the invention, a determination method of determining a two-dimensional or three-dimensional color expectation range, having an arbitrary form and/or shape, for assessing the plausibility of a color formation value obtained in an analytical measurement based on a color formation reaction is disclosed. The determination method comprises steps that, as an example, may be performed in the given order. It shall be noted, however, that a different order may generally also be possible. Further, it may also be possible to perform one or more of the method steps once or repeatedly. Further, it may also be possible to perform two or more of the method steps simultaneously or in a timely overlapping fashion. The determination method may comprise further method steps that are not listed.

The term "an analytical measurement based on a color formation reaction" as used herein is a broad term and is to be given its ordinary and customary meaning to a person of ordinary skill in the art and is not to be limited to a special or customized meaning. The term specifically may refer, without limitation, to a quantitative and/or qualitative determination of at least one analyte in an arbitrary sample or aliquot of bodily fluid by using a color formation reaction. For example, the bodily fluid may comprise one or more of blood, interstitial fluid, urine, saliva or other types of body fluids. The result of the determining of the analyte, as an example, may be a concentration of the analyte and/or the presence or absence of the analyte to be determined. Specifically, as an example, the analytical measurement may be a blood glucose measurement, thus the result of the analytical measurement may for example be a blood glucose concentration. In particular, an analytical measurement result value, such as the concentration of the analyte in the bodily fluid, may be determined by the analytical measurement by using a color formation reaction, such as a color-change reaction in response to a quantitative and/or qualitative presence or absence of the analyte in the bodily fluid.

For example, the bodily fluid may comprise one or more of blood, interstitial fluid, urine, saliva or other types of body fluids. Consequently, the term "sample of bodily fluid" may specifically refer to an arbitrary aliquot part or aliquant part of a biological fluid which directly is a bodily fluid or which is derived from a bodily fluid such as by one or more pre-processing steps, e.g. by centrifugation. As an example, the sample of bodily fluid may be a droplet of a body fluid as gathered from the body of a person, such as a droplet of blood and/or interstitial fluid generated, e.g., by piercing of a skin portion of the person, e.g. with a lancet, a needle or the like. The sample of bodily fluid may also be simply referred to as the sample.

In the analytical measurement based on a color formation reaction specifically an optical test strip is used. The term "optical test strip" as used herein is a broad term and is to be given its ordinary and customary meaning to a person of ordinary skill in the art and is not to be limited to a special or customized meaning. The term specifically may refer, without limitation, to an arbitrary element or device configured for performing a color-change detection reaction. The optical test strip may also be referred to as test strip or test element, wherein all three terms may refer to the same element. The optical test strip may particularly have a reagent test region, also referred to as test region and/or test field, containing at least one test chemical for detecting at least one analyte. Specifically, the test chemical may be configured for performing a color-change, e.g. changing its color, due to a presence of the analyte. The color-change may for example depend on a concentration of the analyte in the sample of bodily fluid. In particular, the reagent test region may have a visually detectable edge, such as a detectable rim and/or border, contrasting the reagent test region from other parts of the optical test strip. The optical test strip, as an example, may comprise at least one substrate, such as at least one carrier, with the at least one test field applied thereto or integrated therein. In particular, the optical test strip may further comprise at least one white area, such as a white field, specifically in a proximity to the reagent test region, for example enclosing or surrounding the test region. In particular, a contrast between the reagent test region and the white area surrounding the reagent test region may be sufficient to allow detecting an edge and/or rim of the reagent test region, specifically visually detecting, for example by using image recognition techniques. Additionally or alternatively, the substrate or carrier itself may be or may comprise the white area. As an example, the at least one carrier may be strip-shaped, thereby rendering the test element a test strip. These test strips are generally widely in use and available. One test strip may carry a single reagent test region or a plurality of test regions having identical or different test chemicals comprised therein.

The term "a color formation value obtained in an analytical measurement based on a color formation reaction" as used herein is a broad term and is to be given its ordinary and customary meaning to a person of ordinary skill in the art and is not to be limited to a special or customized meaning. The term specifically may refer, without limitation, to a numerical indication of the color of the reagent test region of the optical test strip used in the analytical measurement. In particular, the term "color formation value" may refer to an arbitrary numerical indication, such as numerical representation, of the color of the reagent test region of the test strip, specifically resulting from the color-change detection reaction of the test chemical. Specifically, the color numerically indicated by the color formation value may correlate with an analyte concentration of the sample of bodily fluid applied to the respective optical test strip. Thus, as an example, the color and therefore the color formation value may correlate with a blood glucose value and/or blood glucose concentration.

The term "assessing the plausibility" as used herein is a broad term and is to be given its ordinary and customary meaning to a person of ordinary skill in the art and is not to be limited to a special or customized meaning. The term specifically may refer, without limitation, to a process of quantitatively and/or qualitatively determining a credibility and/or probability of an element and/or data. As an example, the plausibility may be assessed by using one or more characteristic parameters and/or properties of the element and/or data. These one or more characteristic parameters and/or properties may, individually or according to a predetermined combination, be compared with one or more conditions. Thus, as an example, the plausibility of the color formation value may be assessed by using one or more characteristic parameters and/or properties of an expected characteristic, specifically by using the color expectation range as will be outlined in further detail below. Specifically, the color formation value may be compared to the color expectation range, e.g. to one or more comparative values, reference values or standard values, wherein the comparison may be a qualitative or quantitative comparison and may result in a binary result such as "plausible" or "not plausible"/"implausible". Additionally or alternatively, however, the comparison may result in a quantitative result, such as a figure indicating a degree of plausibility.

The term "color expectation range" as used herein is a broad term and is to be given its ordinary and customary meaning to a person of ordinary skill in the art and is not to be limited to a special or customized meaning. The term specifically may refer, without limitation, to a continuous and/or discrete scope of a sub-color space, such as to at least part of a possible color space, within which a color is expected and/or predicted to be. Thus, as an example, the color expectation range may be one or more of a two-dimensional or three-dimensional area of a color space comprising at least one of comparative values, reference values and/or standard values. Thus, the color expectation range may be or may comprise a continuous area and/or corridor comprising at least one expected color value. Additionally or alternatively, however, the color expectation range may be or may comprise a conglomerate of discrete expected colors, e.g. comprising discrete comparative values, reference values and/or standard values. The color expectation range, specifically the two-dimensional or three-dimensional color expectation range, has an arbitrary form and/or shape.

Consequently, the term "determination method of determining a color expectation range for assessing the plausibility of a color formation value obtained in an analytical measurement based on a color formation reaction", also simply referred to as a "determination method", as used herein is a broad term and is to be given its ordinary and customary meaning to a person of ordinary skill in the art and is not to be limited to a special or customized meaning. The term specifically may refer, without limitation, to a method of determining the color expectation range as defined above. Specifically, the term may refer to a method by which, i.e. as a result, at least one range defining an expected range of color formation values is determined and/or ascertained, in particular for non-corrupted test strips as will be outlined further below.

The term "training set" as used herein is a broad term and is to be given its ordinary and customary meaning to a person of ordinary skill in the art and is not to be limited to a special or customized meaning. The term specifically may refer, without limitation, to a plurality of elements having known and/or predetermined differences and/or similarities. In particular, the training set may be used for training a trainable model, such as a model that can be further trained and/or updated based on additional information, e.g. gathered from the training set.

The term "training set of optical test strips" may specifically refer, without limitation, to a plurality of optical test strips as defined above. In particular, the training set of optical test strips as provided in step a) comprises a plurality of optical test strips, e.g. of optical test strips identical in construction and/or design. As an example, for the plurality of optical test strips comprised by the training set of optical test strips, at least one characteristic and/or property of one or more of the optical test strips may be known and/or predetermined, such that a difference between one or more of the optical test strips of the training set of optical test strips may be known and/or predetermined.

The term "corrupted optical test strip" as used herein is a broad term and is to be given its ordinary and customary meaning to a person of ordinary skill in the art and is not to be limited to a special or customized meaning. The term specifically may refer, without limitation, to an optical test strip as defined above, wherein at least one function of the optical test strip, such as the color-change detection reaction of the reagent test region, may at least partially be defective and/or altered beyond a tolerable measure. In particular, the corrupted optical test strip may be an optical test strip for which handling and/or storage requirements have been disregarded. Thus, as an example, the corrupted optical test strip, as opposed to the non-corrupted optical test strip, may, when used in an analytical measurement, specifically show a defective and/or incorrect color of the color-change detection reaction, e.g. leading to an invalid analyte concentration. Specifically, the corrupted optical test strip may be a test strip having at least one parameter being out of a predetermined or determinable tolerance range defining non-corrupted optical test strips.

The parameter may be an intrinsic or extrinsic parameter. Therein, an intrinsic parameter may, as an example, refer to a property of the optical test strip itself, whereas an extrinsic parameter may, as an example, refer to a parameter describing a handling of the optical test strip or a use of the optical test strip. Consequently, a corrupted test strip may be corrupted per se and/or may be corrupted by inappropriate handling. The inappropriate handling, as an example, may refer to storage conditions, such that the extrinsic parameter may, as an example, refer to a parameter describing one or more storage conditions, such as a storage temperature and/or a storage time. Additionally or alternatively, the inappropriate handling may refer to a time which has passed since an application of at least one sample of at least one bodily fluid to the optical test strip, specifically to the at least one reagent test region. As an example, the time elapsed between applying the sample to the reagent test region and capturing at least one image comprising at least a part of the at least one reagent test region having the sample applied thereto may have to be in a predetermined tolerance range. Thus, as soon as the time is outside the tolerance range, the optical test strip is corrupted, e.g. as soon as the time which has elapsed since applying the sample has passed a time window within which a tolerable measurement may take place, i.e. within which at least one image is supposed to be captured.

Thus, generally, a corrupted optical test strip may, without limitation, be an optical test strip which is a priori or per se corrupted, and/or a test strip which is being handled incorrectly, e.g. by using the test strip in a non-compliant manner, such as by capturing the respective image at an inappropriate point in time. Therein, two situations of corruption are generally conceivable, which may also occur in combination:
I. the optical test strip being corrupted per se, such as by degradation, faulty material properties, impact of detrimental environmental effects, such as humidity, expiry of shelf life or the like; and/or
II. the optical test strip being corrupted by inappropriate handling and/or use, such as by being used in a measurement performed with one or more measurement parameters out of at least one tolerance range, such as by capturing at least one image comprising at least a part of at least one test region of the optical test strip at an inappropriate point in time, such as outside a tolerance range of times, e.g. with a time elapsed between sample application and capturing of the image being greater than a maximum delay, and/or due to a pre-use of the optical test strip being dedicated for single-use only.

The term "non-corrupted optical test strip" as used herein is a broad term and is to be given its ordinary and customary meaning to a person of ordinary skill in the art and is not to be limited to a special or customized meaning. The term specifically may refer, without limitation, to an optical test strip as defined above, wherein the integrity of the optical test strip has not been jeopardized and/or tampered with. In particular, the non-corrupted optical test strip may be an optical test strip for which handling and/or storage requirements have been regarded. Specifically, the term non-corrupted optical test strip may refer to an optical test strip as defined above, wherein the optical test strip is neither a priori nor per se corrupted, and/or that has been handled correctly, i.e. in a compliant manner, such as by capturing the respective image at an appropriate point in time. Specifically, the non-corrupted optical test strip may be or may comprise an optical test strip that has been handled correctly and is neither a priori nor per se corrupted.

As an example, the color expectation range determined in the determination method may be or may comprise at least one range defining an expected range of color formation values for non-corrupted optical test strips. Specifically, the color expectation range may, as an example, not comprise, i.e. skip or leave out, color formation values of the training set of color formation values for corrupted optical test strips, i.e. for corrupt measurements. Thus, the color expectation range may skip and/or leave out color formation values expected for corrupted optical test strips.

The term "training set of samples of bodily fluids" as used herein is a broad term and is to be given its ordinary and customary meaning to a person of ordinary skill in the art and is not to be limited to a special or customized meaning. The term specifically may refer, without limitation, to a plurality of samples having at least one known and/or predetermined analyte concentration, e.g. determined in a laboratory environment. In particular, the training set of samples of bodily fluids may comprise a plurality of samples of bodily fluids as defined above, wherein for one or more of the plurality of samples the quantitative and/or qualitative analytical measurement result value, such as the concentration of at least one analyte within the sample, is known. Specifically, the number of samples comprised in the training set of samples may differ from the number of optical test strips in the training set of optical test strips. Alternatively, however, the number of samples in the training set of samples may equal the number of optical test strips in the training set of optical test strips.

The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smartphone. Additionally or alternatively, the mobile device may also refer to a tablet computer or another type of portable computer having at least one camera. Further, as will be outlined below, the mobile device may optionally comprise further elements, such as for example one or more processors.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip.

Consequently, the term "training set of images" may specifically relate to a plurality of images, i.e. to a plurality of image data recorded by using a camera, e.g. the camera as defined above. In particular, the training set of images may refer to a plurality of images, e.g. a stack of digital images, of at least one part of one or more of the reagent test regions of the training set of optical test strips. As an example, the training set of images may comprise images of each of the reagent test regions of the training set of optical test strips, wherein, for example, one or more of the samples of bodily fluid may have been applied to the reagent test regions, e.g. before image capturing. Specifically, the training set of images may be used for determining a training set of color formation values of the reagent test regions of the training set of optical test strips, such as a plurality of color formation values for the training set of optical test strips. One or more of the images of the training set of images may be of more than one reagent test region. Thus, as an example, the number of images of the training set of images may differ from the number of optical test strips in the training set of optical test strips. However, equal numbers of images in the training set of images and of optical test strips in the training set of optical test strips may also be possible. Specifically, as an example, the training set of images may for example comprise separate images for each of the reagent test regions of the training set of optical test strips.

The camera, besides the at least one camera chip or imaging chip, may comprise further elements, such as one or more optical elements, e.g. one or more lenses. As an example, the camera may be a fix-focus camera, having at least one lens, which is fixedly adjusted with respect to the camera. Alternatively, however, the camera may also comprise one or more variable lenses, which may be adjusted, automatically or manually. The invention specifically shall be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B, for example also referred to as color channels. A larger number of color values, is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

The term "processor" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math coprocessor or a numeric coprocessor, a plurality of registers, specifically registers configured for supplying operands to the ALU and storing results of operations, and a memory, such as an L1 and L2 cache memory. In particular, the processor may be a multicore processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like.

The processor, specifically the processor which may be used in step d) of the determination method, may, for example, be a separate processor, such as a stand-alone processor or a processor integrated into a computer or computer network, separate from the mobile device. Alternatively, however, the processor may be integrated into the mobile device used in step c) for capturing the training set of images. Thus, specifically, the processor may be a processor of the mobile device.

As an example, the corrupted optical test strip may be corrupted by one or both of a previous appliance of a fluid sample, such as a sample of bodily fluid, and a previous exposure to at least one corruptive environment, e.g. for more than 10 minutes, specifically for more than 2 hours, more specifically for more than 1 day. In particular, the optical test strip may be corrupted, i.e. considered to be corrupted, by having previously had a fluid sample applied, such as by re-dosing and/or double dosing the optical test strip. Specifically, a pre-used and/or re-used optical test strip may be considered to be a corrupted optical test strip. Additionally or alternatively, the test strip may be considered to be corrupted in case of a previous exposure to at least one corruptive environment, such as a corruptive environment damaging the optical test strip. Thus, a damaged optical test strip may be considered to be a corrupted optical test strip. In particular, the optical test strip may be corrupted when having been exposed to the corruptive environment for more than 10 minutes, specifically for more than 2 hours, more specifically for more than 1 day.

The corruptive environment, as an example, may be an environment selected from the group consisting of: a humid environment, specifically an environment having a humidity of more than 60%, more specifically a humidity of more than 80%, and a bright environment, specifically an environment having an illuminance of more than 1000 lm/m², more specifically an illuminance of more than 1500 lm/m².

Step d) may further comprise labelling the color formation values of the training set of color formation values with information on whether the respective optical test strip of the training set of optical test strips was corrupted or non-corrupted. The term "labelling" as used herein is a broad term and is to be given its ordinary and customary meaning to a person of ordinary skill in the art and is not to be limited to a special or customized meaning. The term specifically may refer, without limitation, to a process of linking and/or connecting information. Specifically, the information on whether the respective optical test strip was corrupted or non-corrupted, i.e. not corrupted, may be linked and/or connected to the color formation values. The labelling specifically may be taken into consideration in step e). Specifically, the labelling may be considered when deriving the color expectation range from color formation values for non-corrupted optical test strips, i.e. for distinguishing which of the color formation values of the training set of color formation values was determined for non-corrupted optical test strips or, respectively, for corrupted optical test strips.

In step e), the color expectation range may comprise at least 80%, of the color formation values for the non-corrupted optical test strips of the training set of optical test strips. Specifically, in step e), the color expectation range may comprise at least 85% of the color formation values for the non-corrupted optical test strips of the training set of optical test strips. More specifically, in step e), the color expectation range may comprise at least 90% of the color formation values for the non-corrupted optical test strips of the training set of optical test strips. More specifically, in step e), the color expectation range may comprise at least 95% of the color formation values for the non-corrupted optical test strips of the training set of optical test strips. More specifically, in step e), the color expectation range may comprise at least 97% of the color formation values for the non-corrupted optical test strips of the training set of optical test strips. More specifically, in step e), the color expectation range may comprise at least 99% of the color formation values for the non-corrupted optical test strips of the training set of optical test strips.

As an example, the color expectation range may be or may comprise at least one polygon. Specifically, the color expectation range may be or may comprise a two-dimensional polygon, the edges of which may correspond to the color formation values in a two-dimensional color space, such as in a color plane of at least two colors. Additionally or alternatively, the color expectation range may be or may comprise a three-dimensional polyhedron, the edges of which may correspond to the color formation values in a three-dimensional color space. Further additionally or alternatively, instead of the edges corresponding to the color formation values, the edges may be spaced apart from the color formation values, such that at least 80%, specifically at least 85%, more specifically at least 90%, more specifically at least 95%, more specifically at least 97%, more specifically at least 99%, of the color formation values corresponding to the non-corrupted optical test strips of the training set of optical test strips may be enclosed by the polygon and/or polyhedron.

In particular, the deriving in step e) may comprise determining an envelope comprising at least 80%, specifically at least 85%, more specifically at least 90%, more specifically at least 95%, more specifically at least 97%, more specifically at least 99%, of the color formation values for the non-corrupted optical test strips of the training set of optical test strips and further expanding the envelope by a predetermined safety factor.

The term "envelope" as used herein is a broad term and is to be given its ordinary and customary meaning to a person or ordinary skill in the art and is not to be limited to a special or customized meaning. The term specifically may refer, without limitation, to an element and/or entity enclosing and/or covering at least one set of data. In particular, the envelope may enclose, i.e. in at least one color plane and/or color space, at least 80%, specifically at least 85%, more specifically at least 90%, more specifically at least 95%, more specifically at least 97%, more specifically at least 99%, of the color formation values for the non-corrupted optical test strips of the training set of optical test strips, wherein the envelope may be mathematically and/or graphically determined.

In particular, step e) may comprise, specifically in a subsequent step, expanding the envelope by a predetermined safety factor. The safety factor may specifically be predetermined and/or pre-set, such as a safety factor taking into consideration the size and/or volume of the envelope. As an example, the safety factor may be or may comprise a function dependent on the envelope, i.e. on the size and/or volume of the envelope. As an example, the envelope may be expanded such that a size and/or volume of the expanded envelope may exceed the size and/or volume of the envelope by at least a factor of 1.1, specifically by at least 1.2, more specifically by at least 1.5. As an example, the envelope may be expanded such that 99% or even 100% of the color formation values determined for the non-corrupted optical test strips are enclosed. Additionally or alternatively, the safety factor may take into consideration a deviation of the distribution of the color formation values, such as the standard deviation σ of the color formation values for the non-corrupted optical test strips of the training set of optical test strips. Thus, as an example, the envelope may be expanded by a factor to span a region of at least 4σ, preferably of at least 5σ, more preferably of at least 6σ.

The expansion may specifically be an equally distributed expansion, such as an even and/or uniform expansion of the envelope. As an example, a uniform expansion of the envelope may be performed in case the color formation values are equally distributed within the envelope. An unevenly distributed expansion, however, is also possible. Specifically, the envelope may be non-uniformly and/or unevenly expanded, i.e. based and/or depending on a weighted distribution of the color formation values within the envelope.

Step e) may specifically comprise using at least one machine-learning algorithm, specifically by training a trainable model by using the training set of color formation values. The term "machine-learning algorithm" as used herein is a broad term and is to be given its ordinary and customary meaning to a person of ordinary skill in the art and is not to be limited to a special or customized meaning. The term specifically may refer, without limitation, to a mathematical model being trainable by using records of training data, such as comprising training input data and corresponding training output data. In particular, the training output data of the record of training data may be the result that is expected to be produced by the machine-learning algorithm when being given the training input data of the same record of training data as input. As an example, the deviation between this expected result and the actual result produced by the algorithm may be observed and rated by means of a "loss function". This loss function may be used as a feedback for adjusting the parameters of the internal processing chain of the machine-learning algorithm. The machine-learning algorithm may comprise decision trees, naive bayes classifications, nearest neighbors, neural networks, convolutional neural networks, generative adversarial net-works, support vector machines, linear regression, logistic regression, random forest and/or gradient boosting algorithms. As an example, the machine-learning algorithm may be trained by using the training set of color formation values as input data and the corresponding information on the respective optical test strip, i.e. whether the optical test strip is a corrupted optical test strip or a non-corrupted optical test strip, as output data. Specifically, the machine-learning algorithm may be or may comprise a trainable color expectation range model, i.e. describing a shape and/or form of the color expectation range, wherein the feedback for adjusting the parameters of the internal processing chain of the model may specifically be based on a quantitative or qualitative determination whether the color formation values corresponding to non-corrupted optical test strips are within the color expectation range. Other forms of feedback may be possible.

In step d) the color formation values are specifically determined for at least two color channels, such as for at least two color channels selected from the group consisting of: a green color channel (G), a blue color channel (B) and a red color channel (R). In particular, in step e) the color expectation range is derived for the at least two color channels for which the color formation values are determined in step d).

Further, the method may comprise step f) of attaching at least one optical test strip of the training set of optical test strips to a color reference card comprising a plurality of color reference fields having known reference color values. In particular, step f) may be performed before step c). Furthermore, at least one image of the set of images captured in step c) may further show at least part of the color reference card, specifically one or more of the color reference fields. The term "color reference card" as used herein is a broad term and is to be given its ordinary and customary meaning to a person of ordinary skill in the art and is not to be limited to a special or customized meaning. The term specifically may refer, without limitation, to an arbitrary item having, disposed therein or disposed thereon, such as on at least one surface, the plurality of color reference fields having known color properties or optical properties, such as having a plurality of colored fields having known reference color values. Further, the color reference card may comprise a plurality of gray reference fields having known gray levels. As an example, the color reference card may be a flat card comprising at least one substrate having, on at least one surface and/or disposed therein, the plurality of color reference fields having known color values and the plurality of gray reference fields having known gray levels. The substrate, specifically, may have a flat surface with the color reference fields and the gray reference fields disposed thereon. The substrate, as an example, may be or may comprise one or more of a paper substrate, a cardboard substrate, a plastic substrate, a ceramic substrate or a metal substrate. Laminate substrates are also possible. The substrate, as an example, may be sheet-like or flexible. It shall be noted, however, that the substrate may also be implemented into an article of use, such as into a wall of a box, a vial, a container, a medical consumable, such as a test strip, or the like. The color reference card may also fully or partially be integrated into the optical test strip. The at least one image of at least the part of the reagent test region of the optical test strip may fully or partially comprise an image of at least one part of the color reference card.

In a further aspect of the invention, a measurement method of performing an analytical measurement based on a color formation reaction by using a mobile device having a camera and a processor is disclosed. The measurement method comprises steps that, as an example, may be performed in the given order. It shall be noted, however, that a different order may generally also be possible. Further, it may also be possible to perform one or more of the method steps once or repeatedly. Further, it may also be possible to perform two or more of the method steps simultaneously or in a timely overlapping fashion. The measurement method may comprise further method steps that are not listed.

For the definitions of the measurement method, reference may be made to the description of the determination method described above or as will be outlined in further detail below. Specifically, for performing the measurement method, the same types of optical test strips may be used as in the determination method outlined above. Thus, as an example, the optical test strip provided in step i) of the measurement method may be of the same or at least similar type as the plurality of optical test strips of the training set of optical test strips provided in step a) of the determination method as described above or as will be described in more detail below. The sample of bodily fluid, however, may be a sample of bodily fluid from a user, the analyte concentration of which is to be determined and thus, may previously be unknown.

The measurement method may further comprise an intensity check, such as a step of checking whether an intensity of the color formation value is above or below at least one intensity threshold. In particular, in case the color formation value is outside of a predefined intensity range, e.g. below a lower intensity threshold or above an upper intensity threshold, the measurement method may be aborted.

The measurement method may further comprise step viii) of capturing, by using the camera, at least one image of at least a part of the regent test region without having the bodily fluid applied thereto. In particular, step viii) may be performed before step ii). Thus, as an example, the measurement method may comprise the capturing of at least one second image, specifically a blank image of the reagent test region without having the sample of bodily fluid applied thereto.

Further, the measurement method may comprise step ix) of attaching the optical test strip to a color reference card comprising a plurality of color reference fields having known reference color values. The color reference card may specifically be of the same type as the color reference card optionally used in the determination method as described above. In particular, step ix) may be performed before step iii), and, optionally, before step ii) of the measurement method, wherein specifically, the image captured in step iii) may further show at least part of the color reference card, specifically one or more of the color reference fields.

The applying in step ii) may further comprise confirming, specifically by a user, that the sample of bodily fluid is or has been applied to the regent test region of the optical test strip. Thus, as an example, the application of step ii) may be or may comprise a confirmation of the user that the sample of bodily fluid has been applied, i.e. by pushing a button and/or other form of confirmation, e.g. by interacting with the mobile device. Specifically, step ii) of the measurement method may comprise prompting a user to perform one or more of applying the sample of bodily fluid to the reagent test region of the optical test strip and confirming application of the sample of bodily fluid to the reagent test regions of the optical test strip. In particular, when performing step ii), the user may be prompted to apply the sample of bodily fluid and/or the user may be prompted to confirm sample application, e.g. by providing corresponding instructions on a display of the mobile device and/or as audio instructions.

In a further aspect of the invention, a determination system is disclosed for determining a two-dimensional or three dimensional color expectation range, having an arbitrary form and/or shape, for assessing the plausibility of a color formation value obtained in an analytical measurement based on a color formation reaction.

The processor of the determination system may, for example, be a separate processor, such as a stand-alone processor or a processor integrated into a computer or computer network, separate from the at least one mobile device. Alternatively, however, the processor may be integrated into the mobile device.

The determination system may further comprise:
E) at least one color reference card configured for having the optical test strip releasably attached thereto, the color reference card comprising a plurality of color reference fields having known reference color values, wherein the images of the training set of images further show at least part of the color reference card, specifically one or more of the color reference fields.

For most of the definitions of the determination system, reference may be made to the description of the determination method as described above or as will be outlined in further detail below. In particular, the determination system may be configured for performing the determination method as described herein. In particular, the determination system may be configured for performing at least steps d) and e) of the determination method as described herein.

Further disclosed and proposed herein is a computer program comprising instructions which, when the program is executed by a determination system, specifically by the determination system as described herein, cause the determination system to carry out at least steps d) and e) of the determination method as also described herein. Thus, specifically the computer program may comprise computer-executable instructions for performing the determination method according to the present invention in one or more of the embodiments enclosed herein when the instructions are executed on a determination system, i.e. on the at least one processor of the determination system, for example integrated into a computer or computer network. Specifically, the computer program may be stored on a computer-readable data carrier and/or on a computer-readable storage medium.

Thus, further disclosed and proposed herein is a computer-readable storage medium comprising instructions which, when executed by a determination system, specifically by the determination system as described herein, cause the determination system to carry out at least steps d) and e) of the determination method as also described herein.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Further disclosed and proposed herein is a computer program product having program code means, in order to perform the determination method according to the present invention in one or more of the embodiments enclosed herein when the program is executed on a determination system, i.e. on the at least one processor of the determination system, for example integrated into a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the determination method according to one or more of the embodiments disclosed herein.

Further disclosed and proposed herein is a computer program product with program code means stored on a machine-readable carrier, in order to perform the determination method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Furthermore, disclosed and proposed herein is a modulated data signal which contains instructions readable by a computer system or computer network, for performing the determination method according to one or more of the embodiments disclosed herein.

In a further aspect of the invention, a mobile device having at least one camera and at least one processor is disclosed. The mobile device is configured for performing at least steps iv) to vii) of the measurement method as described herein. Thus, for definitions of terms reference is made to the description above, specifically with regard to the measurement method, as described herein.

Further disclosed and proposed herein is a computer program comprising instructions which, when the program is executed by a mobile device having a camera and a processor, specifically by the mobile device as described herein, cause the mobile device to carry out at least steps iv) to vii) of the measurement method. Thus, specifically, the computer program may comprise computer-executable instructions for performing the measurement method according to the present invention in one or more of the embodiments enclosed herein when the instructions are executed on the processor of the mobile device. Specifically, the computer program may be stored on a computer-readable data carrier and/or on a computer-readable storage medium.

Thus, further disclosed and proposed herein is a computer-readable storage medium comprising instructions which, when executed by a mobile device having a camera and a processor, specifically by the mobile device as described herein, cause the mobile device to carry out at least steps iv) to vii) of the measurement method as also described herein.

Further disclosed and proposed herein is a computer program product having program code means, in order to perform the measurement method according to the present invention in one or more of the embodiments enclosed herein when the program is executed on a mobile device, i.e. on the at least one processor of the mobile device, for example integrated into a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the measurement method according to one or more of the embodiments disclosed herein.

Further disclosed and proposed herein is a computer program product with program code means stored on a machine-readable carrier, in order to perform the measurement method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Furthermore, disclosed and proposed herein is a modulated data signal which contains instructions readable by a computer system or computer network, for performing the measurement method according to one or more of the embodiments disclosed herein.

In a further aspect of the invention, a kit for determining the concentration of at least one analyte in a sample of a bodily fluid, specifically a sample of bodily fluid of a user, is disclosed. The kit comprises the mobile device as described above, specifically the mobile device being configured for performing at least steps iv) to vii) of the measurement method as described herein. The kit further comprises at least one optical test strip having at least one reagent test region.

The methods and devices according to the present invention provide a large number of advantages over similar methods and devices known in the art. Specifically, compared to methods and devices known in the art, the methods and devices as described herein may increase measurement safety. Specifically, measurement safety may be increased by providing an effective fail safe mechanism, for example, due to the color formation value having to be within the color expectation range, in particular when performing the measurement method, in order for the concentration of the analyte to be determined. Thus, specifically, the provided methods and devices may increase measurement safety, since not all and any measured color value is converted into an analyte concentration, e.g. into a blood glucose value. Instead, the present methods and devices may allow for a detection of even subtle color changes and/or shifts that may be introduced by user handling, e.g. by re-dosing and/or double dosing of the test strip and/or general test strip re-use. Furthermore, the proposed methods and devices may also allow for detecting systemic or sporadic errors, such as systemic or sporadic handling errors, e.g. storing the optical test strips outside of a dedicated protection vial for example in a humid or bright environment. Furthermore, measurement safety and accuracy may be improved by the proposed methods and devices compared to known methods and devices. The methods may specifically comprise preventing the determining of the concentration of the analyte in the bodily fluid if the plausibility assessment may not be fulfilled. Therefore, false and/or biased results of the analytical measurement may become more unlikely.

Furthermore, the proposed methods and devices may allow for an increased user handling and/or improved user friendliness of analytical measurement, i.e. by allowing a safe analytical measurement to be performed by only capturing one image, instead of capturing at least two images, such as for example capturing one image before sample application and one image after sample application. Specifically, the overall time necessary for performing the analytical measurement may be decreased compared to known methods and devices.

Further optional features and embodiments will be disclosed in more detail in the subsequent description of embodiments, preferably in conjunction with the dependent claims.

The scope of the invention is defined in the appended claims.

The embodiments are schematically depicted in the Figures. Therein, identical reference numbers in these Figures refer to identical or functionally comparable elements.

In the Figures:
- Figure 1: shows an embodiment of a determination system and an embodiment of a kit;
- Figure 2: shows a graphical illustration of an embodiment of a determination method;
- Figures 3 and 4: show embodiments of color expectation ranges;
- Figures 5 and 6: show graphical illustrations of different embodiments of a measurement method; and
- Figure 7: shows a graphical illustration of a diagram indicating a relationship between actual blood glucose values and determined blood glucose values by using both common methods and systems and using present methods and systems for the determination of blood glucose concentrations.

### Detailed description of the embodiments

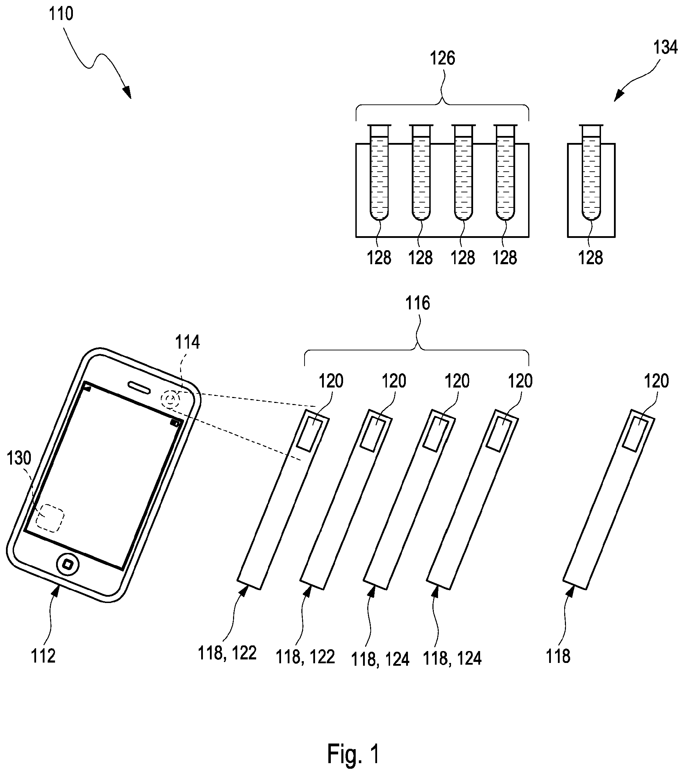

In Figure 1, an embodiment of a determination system 110 is illustrated. The determination system 110 comprises at least one mobile device 112 having at least one camera 114. Further, the determination system 110 comprises a training set of optical test strips 116. The training set of optical test strips 116 comprises a plurality of optical test trips 118, each optical test strip 118 having a reagent test region 120. At least two of the optical test strips 118 are non-corrupted and at least two of the optical test strips 118 are corrupted. Thus, the training set of optical test strips 116 comprises at least two non-corrupted optical test strips 122 and at least two corrupted optical test strips 124. Further, the determination system 110 comprises a training set of samples of body fluids 126 comprising a plurality of samples of bodily fluids 128. In particular, for each of the samples of bodily fluids 128 and the training set of samples of body fluid 126 and analyte concentration, e.g. a glucose concentration, may be known. Furthermore, the determination system 110 comprises at least one processor 130. The processor 130 of the determination system 110 may, for example, be a separate processor 130. Alternatively however, and as illustrated in Figure 1, the processor 130 may be integrated into the mobile device 112, such that the processor 130 may be a processor 130 of the mobile device 112.

In the determination system 110 the processor 130 is configured for retrieving a training set of images comprising images captured with the camera 114 of the mobile device 112. In particular, the images of the training set of images may be images of at least one part of each of the reagent test regions 120 of the optical test strips 118 of the training set of optical test strips 116 having applied thereto at least one sample of bodily fluid 128 of the training set of samples of bodily fluids 126. Further, in the determination system 110, the process of 130 is configured for determining a training set of color formation values from the images of the training set of images. The training set of color formation values comprises color formation values of at least one color channel for the color formation of the reagent test region 120 each of the optical test strips 118 of the training set of optical test strips 116. Furthermore, in the determination system 110, the processor 130 may be configured for deriving at least one color expectation range 132 (not illustrated in Figure 1) for the at least one color channel from the training set of color formation values, wherein the color expectation range 132 defines an expected range of color formation values for non-corrupted optical test strips 122.

Figure 1 also illustrates an embodiment of a kit 134 configured for determining the concentration of at least one analyte in a sample of bodily fluid 128. The sample of bodily fluid 128 may specifically comprise a bodily fluid of a user, such as a sample having an unknown analyte concentration. In Figure 1, such a sample is exemplary illustrated on the far right of the figure. The kit 134 comprises at least one optical test strip 118 having at least one reagent test region 120. In Figure 1, such an optical test strip 118, i.e. a singular optical test strip 118, is exemplary illustrated on the far right of the figure. Further, the kit 134 comprises a mobile device 112 having at least one camera 114 and at least one processor 130. As an example, the mobile device 112 of the kit 134 may be the same mobile device 112 of the determination system 110. Alternatively however, the kit 134 and the determination system 110 each comprise their own mobile device 114, i.e. distinct and separate mobile devices 112.

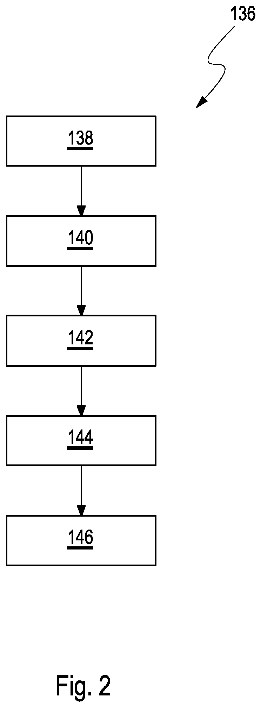

The determination system 110 may specifically be configured for at least partially performing a determination method 136. An exemplary embodiment of the determination method 136 is shown in Figure 2. The determination method 136 is configured for determining a color expectation range 132 processing the plausibility of a color formation value obtained in an analytical measurement based on a color formation reaction. The determination method 136 comprises the following steps:
a) (denoted with reference number 138) providing a training set of optical test strips 116, each optical test strip 118 having a reagent test region 120, wherein at least two of the optical test strips are non-corrupted 122 and wherein at least two of the optical test strips are corrupted 124;
b) (denoted with reference number 140) providing a training set of samples of bodily fluids 126 and applying at least one of the samples of bodily fluid 128 to the reagent test region 120 of each optical test strip 118 of the training set of optical test strips 116;
c) (denoted with reference number 142) capturing, by at least one mobile device 112 having at least one camera 114, a training set of images, the training set of images comprising images of at least one part of one or more of the reagent test regions 120 of the training set of optical test strips 116 having the sample of bodily fluid 128 applied thereto;
d) (denoted with reference number 144) determining, specifically by using at least one processor 130, more specifically a processor 130 of the mobile device 112, a training set of color formation values from the images of the training set of images, comprising color formation values of at least two color channels for the color formation of the reagent test region 120 of each of the optical test strips 118 of the training set of optical test strips 116; and
e) (denoted with reference number 146) deriving the color expectation range 132 for the at least two color channels from the training set of color formation values, the color expectation range 132 defining an expected range of color formation values for non-corrupted optical test strips 122.

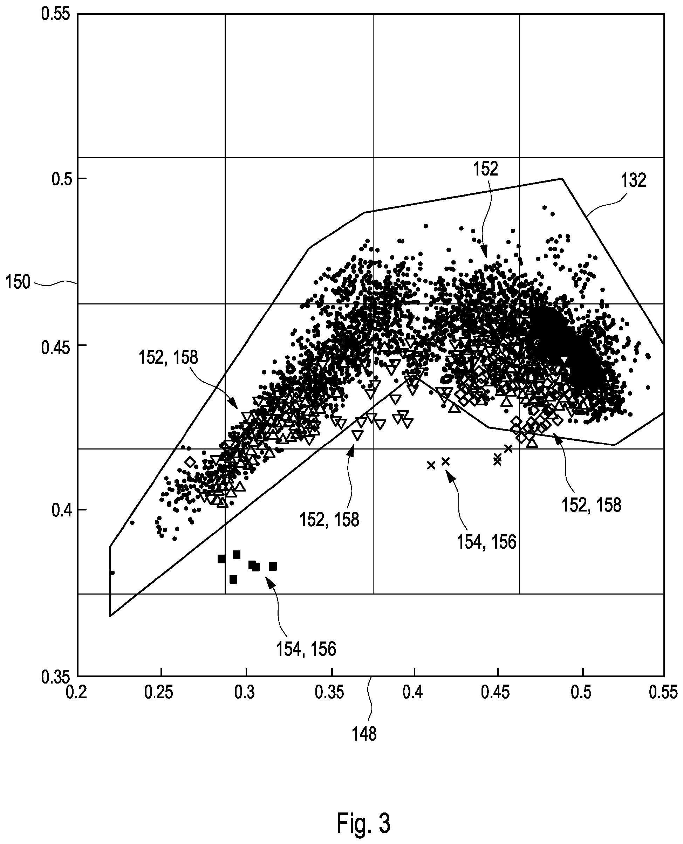

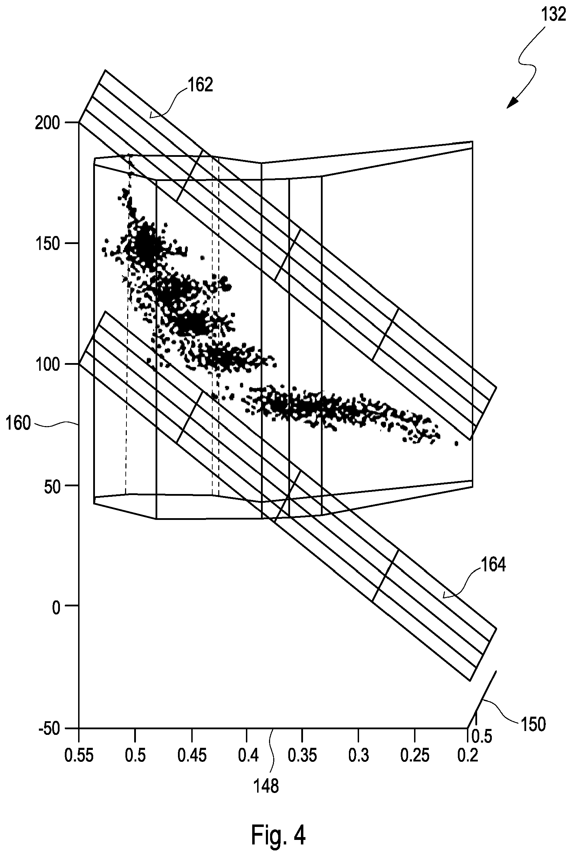

In Figures 3 and 4, exemplary embodiments of color expectation ranges 132 are shown. As illustrated in Figure 3, the color expectation range 132 may, for example, be or may comprise a polygon, such as a two-dimensional polygon in a color plane. For example, on a horizontal axis the color plane may show color values in a red color channel 148, such as a relative red color channel, wherein on a vertical axis the color plane may show color values in a green color channel 150, such as a relative green color channel. Further, in Figure 3, color formation values of an exemplary training set of color formation values are illustrated. As an example, at least 95% of the color formation values corresponding to the non-corrupted optical test strips 122, i.e. non-corrupted color formation values 152, may be enclosed by the polygon shape of the color expectation range 132, whereas a plurality, preferably a majority or even all, of corrupted color formation values 154 corresponding to corrupted optical test strips 124 may specifically be located outside of the polygon shape of the color expectation range 132.

As an example, corrupted color formation values 154 corresponding to differently corrupted optical test strips 124 may be shown in Figure 3, wherein some of the corrupted color formation values 154 may be re-use corrupted color formation values 156, corrupted due to a re-use of the optical test strip 118, i.e. by double dosing and/or re-dosing.

Re-use, as an example, may happen in case of a second blood application, i.e. even having a significantly different blood glucose concentration. As an example, strong measurement deviations may occur in case of applying a significantly lower blood glucose concentration blood sample. This may also refer to a medically critical case, when an actual low value may not be recognized and instead a higher blood glucose result would be presented. Another example for a re-use case may be an initial underdosing, followed by a second measurement with proper amount of blood.

Further, Figure 3 may illustrate non-corrupted color formation values 152 corresponding to non-corrupted optical test strips 122 that may nevertheless have been handled by a user in a slightly incorrect way, wherein however, the slight incorrect handling has not corrupted the optical test strip 118 leading to healed non-corrupted color formation values 158. Such healed non-corrupted color formation values 158 may be non-corrupted color formation values 152 corresponding to non-corrupted optical test strips 122, wherein an initial corruption was healed. As an example, an initial underdosing may have been immediately corrected, i.e. within a few minutes, specifically within 3 minutes. As another example, the optical test strip 118 may have been stored outside a protective vial as and/or within an open vial at 85% rel. hum. and 23°C for only a short period of time, i.e. up to 72 hours. Such a storage may still provide correct measurement values, i.e. non-corrupted color-formation values 152. However, as an example, in case the optical test strip 118 may have been stored in this environment for more than 72 hours up to 336 hours, normal to high glucose concentrations, i.e. ≥ 100mg/dl, may show only slight measurement deviations, wherein lower glucose concentration samples, i.e. < 100mg/dl, may show strong measurement deviations and thus are considered to be corrupted color formation values 154. Further, as illustrated in Figure 3, not all of the healed non-corrupted color formation values 118 may be enclosed by the color expectation range 132.

Additionally or alternatively, and as exemplarily illustrated in Figure 4, the color expectation range 132 may be or may comprise a three-dimensional form, such as a polyhedron. As an example, the color expectation range 132 may be formed by a polygon in a two-dimensional color space, such as a polygon in the color plane of the red color channel 148 and the green color channel 150, that has been expanded into a third dimension, for example referring to a blue color channel 160 or, alternatively, to an Intensity value, i.e. a value in an intensity dimension, for example an absolute intensity value of the values of the green color channel 150. In particular, the color expectation range 132 may be limited in the direction of the blue color channel 160 by an upper plane 162 as well as by a lower plane 164. Other forms and/or geometries of the color expectation range 132 may be possible.

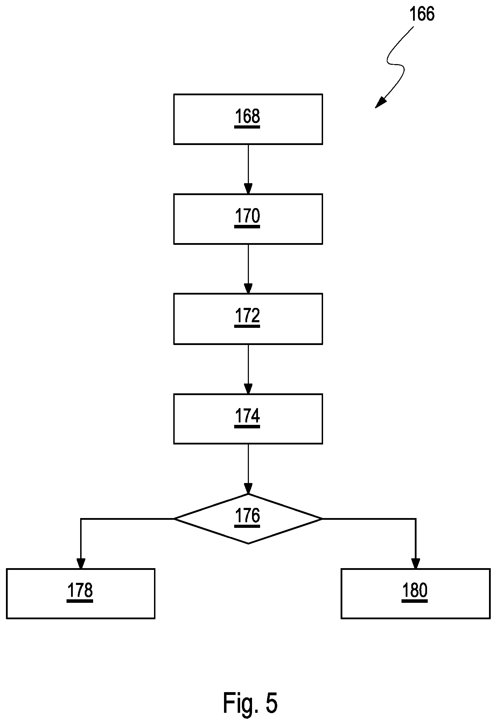

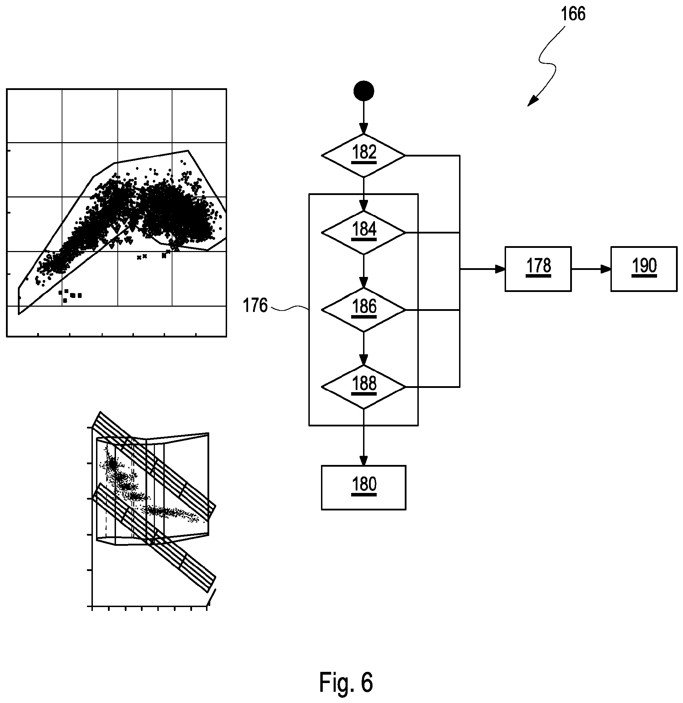

Specifically, the color expectation range 132 may be used for assessing the possibility of a color formation value obtained in an analytical measurement based on the color formation reaction. Such an analytical measurement may be performed by a measurement method 166. In particular, the mobile device 112, specifically the mobile device 112 of the kit 134, may be configured for at least partially performing the measurement method 166. Exemplary embodiments of the measurement method 166 illustrated in Figures 5 and 6. The measurement method 166 comprises the following steps:
i) (denoted with reference number 168) providing at least one optical test strip 118 having at least one reagent test region 120;
ii) (denoted with reference number 170) applying a sample of bodily fluid 128 to the reagent test region 120 of the optical test strip 118;
iii) (denoted with reference number 172) capturing, by using the camera 114, at least one image of at least a part of the reagent test region 120 having the bodily fluid 128 applied thereto;
iv) (denoted with reference number 174) determining, specifically by using the processor 130, a color formation value of at least two color channels for a color formation of the reagent test region 120 by using the image;
v) (denoted with reference number 176) comparing, for the at least two color channels, the color formation value to a color expectation range 132 determined by performing the determination method 136;
vi) (denoted with reference number 178) if the color formation value is outside of the color expectation range 132, considering the color formation value to be not plausible and aborting the measurement method 166; and
vii) (denoted with reference number 180) if the color formation value is inside of the color expectation range, considering the color formation value to be plausible and determining a concentration of analyte in the sample of bodily fluid by using the color formation value.

Another embodiment of the measurement method 166 is illustrated in Figure 6. A starting point may be illustrated by a filled circle at the top of the figure. As an example, the measurement method may further comprise an intensity check 182, such as a step of checking whether an intensity of the color formation value is above or below at least one intensity threshold. In particular, in case the color formation value is outside of a predefined intensity range, e.g. below a lower intensity threshold or above an upper intensity threshold, the measurement method may be aborted. Specifically, in case the color formation value fails the intensity check, the color formation value may be considered not plausible and thus, the measurement method 166 may be aborted. As an example, in case the color expectation range 132 corresponds to the color expectation range 132 illustrated in Figure 4, step v) 176 of the measurement method 166 may specifically comprise separate steps for comparing the color formation value to the color expectation range 132. As an example, as denoted with reference number 184, it may be checked whether the color formation value is above the lower plane 164. Further, as denoted with reference number 186, it may be checked whether the color formation value is below the upper plane 162. Furthermore, as denoted with reference number 188, it may be checked whether the color formation value is within the polygon shape in the color plane defined by the red color channel 148 and the green color channel 150. In case the color formation value fails any of checks 184 to 188, the color formation value may be considered not plausible and the method may be aborted, according to step vi) 178. Further optionally, after aborting the method, the measurement method 166 may comprise showing an error message 190.

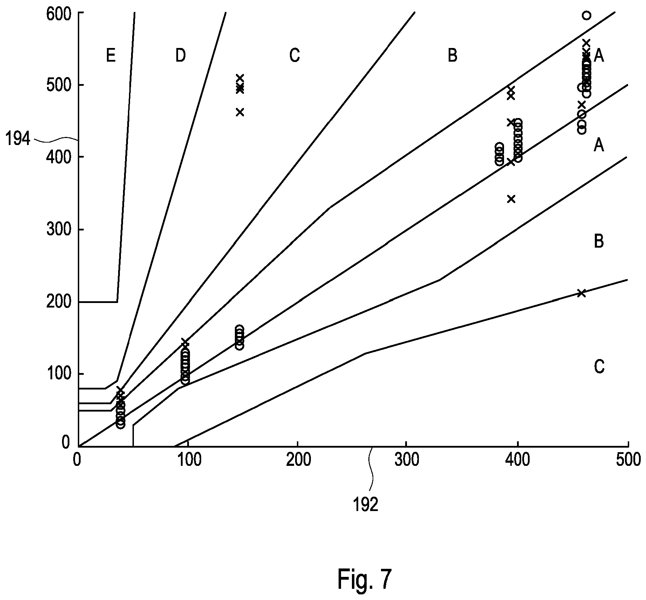

Figure 7 shows a graphical illustration of a diagram indicating a relationship between actual blood glucose values and determined blood glucose values by using both common methods and systems and using present methods and systems for the determination of blood glucose concentrations. In particular, the diagram illustrated in Figure 7 indicates the relationship between the actual blood glucose value in mg/dl 192 and the measured blood glucose value in mg/dl 194. In particular, values indicated by a cross show the relationship for measurements performed by known methods and devices, wherein values indicated with circles show the relationship for measurement performed by using the present methods and devices.

Further, Figure 7 show regions A to E of an Error-Grid-Analysis, specifically regions A to E of the Parkes Error Grid, quantifying clinical accuracy of a determined blood glucose concentration compared to an actual blood glucose concentration. For example blood glucose values within:
a) Region A contains values within 20% of the reference sensor;
b) Region B contains values that are outside of 20% but would not lead to inappropriate treatment;
c) Region C contains values leading to unnecessary treatment;
d) Region D contains values indicating a potentially dangerous failure to detect hypoglycemia or hyperglycemia, and
e) Region E contains values that would confuse treatment of hypoglycemia for hyperglycemia and vice versa.

For more information on the Error-Grid-Analysis reference may be made to Clarke WL, Cox D, Gonder-Frederick LA, Carter W, Pohl SL: Evaluating clinical accuracy of systems for self-monitoring of blood glucose. Diabetes Care 10:622-628,1987.

The measurements illustrated in Figure 7 are based on the same samples, particularly on the same optical test strips 118 having the same samples applied thereto. In particular, the same optical test strips 118 have been used for determining the blood glucose values for both measurements, Table 1 indicates the number of determined blood glucose values for both the measurements performed by using known methods and devices and the measurements performed by using the present methods and devices.

**Table 1: Number of determined blood glucose values for both using known methods and devices and using present methods and devices.**

| Legend | A | B | C | D | E |
|---|---|---|---|---|---|
| number of determined blood glucose values for known methods and devices | 168 | 4 | 5 | 0 | 0 |
| number of determined blood glucose values for present methods and devices | 148 | 1 | 0 | 0 | 0 |

Specifically, as can be seen in the diagram, the values of measurements performed by known methods and devices provide blood glucose values for all measurements, wherein in measurements according to the present invention, no blood glucose values are provided for corrupted optical test strips. In particular, the missing numbers, when using the present methods and devices, are due to a higher amount of aborted measurements, e.g. with a fail safe error.

### List of reference numbers

- 110: determination system
- 112: mobile device
- 114: camera
- 116: training set of optical test strips
- 118: optical test strip
- 120: reagent test region
- 122: non-corrupted optical test strip
- 124: corrupted optical test strip
- 126: training set of samples of bodily fluids
- 128: sample of bodily fluid
- 130: processor
- 132: color expectation range
- 134: kit
- 136: determination method
- 138: step a)
- 140: step b)
- 142: step c)
- 144: step d)
- 146: step e)
- 148: red color channel
- 150: green color channel
- 152: non-corrupted color formation values
- 154: corrupted color formation values
- 156: re-use corrupted color formation values
- 158: healed non-corrupted color formation values
- 160: blue color channel
- 162: upper plane
- 164: lower plane
- 166: measurement method
- 168: step i)
- 170: step ii)
- 172: step iii)
- 174: step iv)
- 176: step v)
- 178: step vi)
- 180: step vii)
- 182: intensity check
- 184: check if color formation value is above lower plane
- 186: check if color formation value is below upper plane
- 188: check if color formation value is within polygon
- 190: show error message
- 192: actual blood glucose value in mg/dl
- 194: measured blood glucose value in mg/dl

## Claims

1. A determination method of determining a two-dimensional or three-dimensional color expectation range (132), having an arbitrary form and/or shape, for assessing the plausibility of a color formation value obtained in an analytical measurement based on a color formation reaction, the method comprising:
a) providing a training set of optical test strips (116), each optical test strip (118) having a reagent test region (120);
b) providing a training set of samples of bodily fluids (126) and applying at least one of the samples of bodily fluid (128) to the reagent test region (120) of each optical test strip (118) of the training set of optical test strips (116);
c) capturing, by at least one mobile device (112) having at least one camera (114), a training set of images, the training set of images comprising images of at least one part of one or more of the reagent test regions (120) of the training set of optical test strips (116) having the sample of bodily fluid (128) applied thereto, wherein at least two of the optical test strips (118) are non-corrupted (122) and wherein at least two of the optical test strips are corrupted (124);
d) determining a training set of color formation values from the images of the training set of images, comprising color formation values of at least two color channels for the color formation of the reagent test region (120) of each of the optical test strips (118) of the training set of optical test strips (116); and
e) deriving the color expectation range (132) for the at least two color channels from the training set of color formation values, the color expectation range (132) defining an expected range of color formation values for non-corrupted optical test strips (122).

2. The determination method according to the preceding claim, wherein the corrupted optical test strip (124) is corrupted by one or both of a previous appliance of a fluid sample and a previous exposure to at least one corruptive environment for more than 10 minutes.

3. The determination method according to the preceding claim, wherein the corruptive environment is an environment selected from the group consisting of: a humid environment and a bright environment.

4. The determination method according to any one of the preceding claims, wherein step d) further comprises labelling the color formation values of the training set of color formation values with information on whether the respective optical test strip (118) of the training set of optical test strips (116) was corrupted or non-corrupted.

5. The determination method according to any one of the preceding claims, wherein in step e) the color expectation range (132) comprises at least 80% of the color formation values for the non-corrupted optical test strips (122) of the training set of optical test strips (116).

6. The determination method according to any one of the preceding claims, wherein the deriving in step e) comprises determining an envelope comprising at least 80%, of the color formation values for the non-corrupted optical test strips (122) of the training set of optical test strips (116) and further expanding the envelope by a predetermined safety factor.

7. The determination method according to any one of the preceding claims, wherein step e) comprises using at least one machine-learning algorithm.

8. A measurement method of performing an analytical measurement based on a color formation reaction by using a mobile device (112) having a camera (114) and a processor (130), the method comprising:
i) providing at least one optical test strip (118) having at least one reagent test region (120);
ii) applying a sample of bodily fluid (128) to the reagent test region (120) of the optical test strip (118);
iii) capturing, by using the camera (114), at least one image of at least a part of the reagent test region (120) having the bodily fluid applied thereto;
iv) determining a color formation value of at least two color channels for a color formation of the reagent test region (120) by using the image;
v) comparing, for the at least two color channels, the color formation value to a two-dimensional or three-dimensional color expectation range (132), having an arbitrary form and/or shape, determined by performing the determination method according to any one of the preceding claims;
vi) if the color formation value is outside of the color expectation range (132), considering the color formation value to be not plausible and aborting the measurement method; and
vii) if the color formation value is inside of the color expectation range (132), considering the color formation value to be plausible and determining a concentration of analyte in the sample of bodily fluid by using the color formation value.

9. The measurement method according to the preceding claim, wherein the method further comprises step viii) of capturing, by using the camera (114), at least one image of at least a part of the regent test region (120) without having the bodily fluid applied thereto, wherein step viii) is performed before step ii).

10. The measurement method according to any one of the two preceding claims, wherein the method further comprises step ix) of attaching the optical test strip (118) to a color reference card comprising a plurality of color reference fields having known reference color values, wherein step ix) is performed before step iii), and wherein the image captured in step iii) further shows at least part of the color reference card.

11. A determination system (110) for determining a two-dimensional or three-dimensional color expectation range (132), having an arbitrary form and/or shape, for assessing the plausibility of a color formation value obtained in an analytical measurement based on a color formation reaction, comprising:
A) at least one mobile device (112) having at least one camera (114);
B) a training set of optical test strips (116), each optical test strip (118) having a reagent test region (120), wherein at least two of the optical test strips are non-corrupted (122) and wherein at least two of the optical test strips are corrupted (124);
C) a training set of samples of bodily fluids (126) comprising a plurality of samples of bodily fluids (128); and
D) at least one processor (130), the processor (130) being configured for
- retrieving a training set of images, the training set of images comprising images, captured with the camera (114), of at least one part of each of the reagent test regions (120) of the training set of optical test strips (116) having the sample of bodily fluid (128) applied thereto;
- determining a training set of color formation values from the images of the training set of images, comprising color formation values of at least two color channels for the color formation of the reagent test region (120) of each of the optical test strips (118) of the training set of optical test strips (116); and
- deriving the color expectation range (132) for the at least two color channels from the training set of color formation values, the color expectation range (132) defining an expected range of color formation values for non-corrupted optical test strips (122).

12. The determination system (110) according to the preceding claim, wherein the determination system (110) further comprises:
E) at least one color reference card configured for having the optical test strip (118) releasably attached thereto, the color reference card comprising a plurality of color reference fields having known reference color values, wherein the images of the training set of images further show at least part of the color reference card.

13. The determination system (110) according to any one of the two preceding claims, wherein the determination system (110) is configured for performing at least steps d) and e) of the determination method according to any one of the preceding claims referring to a determination method.

14. A mobile device (112) having at least one camera (114) and at least one processor (130), the mobile device (112) being configured for performing at least steps iv) to vii) of the measurement method according to any one of the preceding claims referring to a measurement method.

15. A kit (134) for determining the concentration of at least one analyte in a sample of a bodily fluid (128), the kit (134) comprising the mobile device (112) according to the preceding claim, the kit (134) further comprising at least one optical test strip (118) having at least one reagent test region (120).

## Patentansprüche

1. Bestimmungsverfahren zur Bestimmung eines zweidimensionalen oder dreidimensionalen Farberwartungsbereichs (132), der eine beliebige Form und/oder Gestalt aufweist, zur Beurteilung der Plausibilität eines Farbbildungswerts, der in einer analytischen Messung basierend auf einer Farbbildungsreaktion erhalten wird, wobei das Verfahren Folgendes umfasst:
a) Bereitstellen eines Trainingssatzes von optischen Teststreifen (116), wobei jeder optische Teststreifen (118) eine Reagenztestregion (120) aufweist;
b) Bereitstellen eines Trainingssatzes von Körperflüssigkeitsproben (126) und Aufbringen mindestens einer der Körperflüssigkeitsproben (128) auf die Reagenztestregion (120) jedes optischen Teststreifens (118) des Trainingssatzes von optischen Teststreifen (116);
c) Erfassen eines Trainingssatzes von Bildern durch mindestens ein mobiles Gerät (112), das mindestens eine Kamera (114) aufweist, wobei der Trainingssatz von Bildern Bilder von mindestens einem Teil von einer oder mehreren der Reagenztestregionen (120) des Trainingssatzes von optischen Teststreifen (116) umfasst, auf die die Körperflüssigkeitsprobe (128) aufgebracht wurde,
wobei mindestens zwei der optischen Teststreifen (118) nicht beschädigt sind (122) und wobei mindestens zwei der optischen Teststreifen beschädigt sind (124);
d) Bestimmen eines Trainingssatzes von Farbbildungswerten aus den Bildern des Trainingssatzes von Bildern, umfassend Farbbildungswerte von mindestens zwei Farbkanälen für die Farbbildung der Reagenztestregion (120) jedes der optischen Teststreifen (118) des Trainingssatzes von optischen Teststreifen (116); und
e) Ableiten des Farberwartungsbereichs (132) für die mindestens zwei Farbkanäle aus dem Trainingssatz von Farbbildungswerten, wobei der Farberwartungsbereich (132) einen erwarteten Bereich von Farbbildungswerten für nicht beschädigte optische Teststreifen (122) definiert.

2. Bestimmungsverfahren nach dem vorhergehenden Anspruch, wobei der beschädigte optische Teststreifen (124) durch eine vorherige Anwendung einer Flüssigkeitsprobe und/oder ein vorheriges Aussetzen gegenüber mindestens einer beschädigenden Umgebung für mehr als 10 Minuten beschädigt wurde.

3. Bestimmungsverfahren nach dem vorhergehenden Anspruch, wobei die beschädigende Umgebung eine Umgebung ist, die ausgewählt ist aus der Gruppe, bestehend aus: einer feuchten Umgebung und einer hellen Umgebung.

4. Bestimmungsverfahren nach einem der vorhergehenden Ansprüche, wobei Schritt d) ferner das Kennzeichnen der Farbbildungswerte des Trainingssatzes von Farbbildungswerten mit Informationen darüber, ob der jeweilige optische Teststreifen (118) des Trainingssatzes von optischen Teststreifen (116) beschädigt oder nicht beschädigt war, umfasst.

5. Bestimmungsverfahren nach einem der vorhergehenden Ansprüche, wobei in Schritt e) der Farberwartungsbereich (132) mindestens 80 % der Farbbildungswerte für die nicht beschädigten optischen Teststreifen (122) des Trainingssatzes von optischen Teststreifen (116) umfasst.

6. Bestimmungsverfahren nach einem der vorhergehenden Ansprüche, wobei das Ableiten in Schritt e) das Bestimmen einer Hüllkurve, die mindestens 80 % der Farbbildungswerte für die nicht beschädigten optischen Teststreifen (122) des Trainingssatzes von optischen Teststreifen (116) umfasst, und ferner das Erweitern der Hüllkurve um einen vorbestimmten Sicherheitsfaktor umfasst.

7. Bestimmungsverfahren nach einem der vorhergehenden Ansprüche, wobei Schritt e) die Verwendung mindestens eines Algorithmus für maschinelles Lernen umfasst.

8. Messverfahren zur Durchführung einer analytischen Messung auf der Basis einer Farbbildungsreaktion unter Verwendung eines mobilen Geräts (112) mit einer Kamera (114) und einem Prozessor (130), wobei das Verfahren Folgendes umfasst:
i) Bereitstellen mindestens eines optischen Teststreifens (118) mit mindestens einer Reagenztestregion (120);
ii) Aufbringen einer Körperflüssigkeitsprobe (128) auf die Reagenztestregion (120) des optischen Teststreifens (118);
iii) Erfassen mindestens eines Bildes von mindestens einem Teil der Reagenztestregion (120), auf die die Körperflüssigkeit aufgebracht wurde, unter Verwendung der Kamera (114);
iv) Bestimmen eines Farbbildungswertes von mindestens zwei Farbkanälen für eine Farbbildung der Reagenztestregion (120) unter Verwendung des Bildes;
v) Vergleichen des Farbbildungswertes mit einem zweidimensionalen oder dreidimensionalen Farberwartungsbereich (132), der eine beliebige Form und/oder Gestalt aufweist und durch Durchführen des Bestimmungsverfahrens nach einem der vorhergehenden Ansprüche bestimmt wird, für die mindestens zwei Farbkanäle;
vi) wenn der Farbbildungswert außerhalb des Farberwartungsbereichs (132) liegt, Betrachten des Farbbildungswertes als nicht plausibel und Abbrechen des Messverfahrens; und
vii) wenn der Farbbildungswert innerhalb des Farberwartungsbereichs (132) liegt, Betrachten des Farbbildungswertes als plausibel und Bestimmen einer Konzentration des Analyten in der Körperflüssigkeitsprobe unter Verwendung des Farbbildungswertes.

9. Messverfahren nach dem vorhergehenden Anspruch, wobei das Verfahren ferner Schritt viii) des Erfassens mindestens eines Bildes von mindestens einem Teil der Reagenztestregion (120), auf die keine Körperflüssigkeit aufgebracht wurde, unter Verwendung der Kamera (114) umfasst, wobei Schritt viii) vor Schritt ii) durchgeführt wird.

10. Messverfahren nach einem der zwei vorhergehenden Ansprüche, wobei das Verfahren ferner Schritt ix) des Anbringens des optischen Teststreifens (118) an einer Farbreferenzkarte umfasst, die eine Vielzahl von Farbreferenzfeldern mit bekannten Referenzfarbwerten umfasst, wobei Schritt ix) vor Schritt iii) durchgeführt wird und wobei das in Schritt iii) erfasste Bild ferner mindestens einen Teil der Farbreferenzkarte zeigt.

11. Bestimmungssystem (110) zur Bestimmung eines zweidimensionalen oder dreidimensionalen Farberwartungsbereichs (132), der eine beliebige Gestalt und/oder Form aufweist, zur Beurteilung der Plausibilität eines Farbbildungswerts, der in einer analytischen Messung basierend auf einer Farbbildungsreaktion erhalten wird, umfassend:
A) mindestens ein mobiles Gerät (112) mit mindestens einer Kamera (114);
B) einen Trainingssatz von optischen Teststreifen (116), wobei jeder optische Teststreifen (118) eine Reagenztestregion (120) aufweist, wobei mindestens zwei der optischen Teststreifen nicht beschädigt sind (122) und wobei mindestens zwei der optischen Teststreifen beschädigt sind (124);
C) einen Trainingssatz von Körperflüssigkeitsproben (126), der eine Vielzahl von Körperflüssigkeitsproben (128) umfasst; und
D) mindestens einen Prozessor (130), wobei der Prozessor (130) zu Folgendem konfiguriert ist:
- Abrufen eines Trainingssatzes von Bildern, wobei der Trainingssatz von Bildern mit der Kamera (114) erfasste Bilder von mindestens einem Teil von jeder der Reagenztestregionen (120) des Trainingssatzes von optischen Teststreifen (116) umfasst, auf die die Körperflüssigkeitsprobe (128) aufgebracht wurde;
- Bestimmen eines Trainingssatzes von Farbbildungswerten aus den Bildern des Trainingssatzes von Bildern, umfassend Farbbildungswerte von mindestens zwei Farbkanälen für die Farbbildung der Reagenztestregion (120) jedes der optischen Teststreifen (118) des Trainingssatzes von optischen Teststreifen (116); und
- Ableiten des Farberwartungsbereichs (132) für die mindestens zwei Farbkanäle aus dem Trainingssatz von Farbbildungswerten, wobei der Farberwartungsbereich (132) einen erwarteten Bereich von Farbbildungswerten für nicht beschädigte optische Teststreifen (122) definiert.

12. Bestimmungssystem (110) nach dem vorhergehenden Anspruch, wobei das Bestimmungssystem (110) ferner Folgendes umfasst:
E) mindestens eine Farbreferenzkarte, die so konfiguriert ist, dass der optische Teststreifen (118) lösbar daran angebracht ist, wobei die Farbreferenzkarte eine Vielzahl von Farbreferenzfeldern mit bekannten Referenzfarbwerten umfasst, wobei die Bilder des Trainingssatzes von Bildern ferner mindestens einen Teil der Farbreferenzkarte zeigen.

13. Bestimmungssystem (110) nach einem der zwei vorhergehenden Ansprüche, wobei das Bestimmungssystem (110) zum Durchführen mindestens der Schritte d) und e) des Bestimmungsverfahrens nach einem der vorhergehenden Ansprüche, die sich auf ein Bestimmungsverfahren beziehen, konfiguriert ist.

14. Mobiles Gerät (112) mit mindestens einer Kamera (114) und mindestens einem Prozessor (130), wobei das mobile Gerät (112) zum Durchführen mindestens der Schritte iv) bis vii) des Messverfahrens nach einem der vorhergehenden Ansprüche, die sich auf ein Messverfahren beziehen, konfiguriert ist.

15. Kit (134) zum Bestimmen der Konzentration von mindestens einem Analyten in einer Körperflüssigkeitsprobe (128), wobei der Kit (134) das mobile Gerät (112) nach dem vorhergehenden Anspruch umfasst, wobei der Kit (134) ferner mindestens einen optischen Teststreifen (118) mit mindestens einer Reagenztestregion (120) umfasst.

## Revendications

1. Procédé de détermination permettant de déterminer un intervalle d'attente de couleur (132) bidimensionnel ou tridimensionnel, ayant une forme et/ou un contour arbitraire, pour évaluer la plausibilité d'une valeur de formation de couleur obtenue dans une mesure analytique basée sur une réaction de formation de couleur, le procédé comprenant :
a) la fourniture d'un ensemble d'entraînement de bandelettes de test optiques (116), chaque bandelette de test optique (118) ayant une région de test de réactifs (120) ;
b) la fourniture d'un ensemble d'entraînement d'échantillons de fluides corporels (126) et l'application d'au moins l'un des échantillons de fluide corporel (128) sur la région de test de réactifs (120) de chaque bandelette de test optique (118) de l'ensemble d'entraînement de bandelettes de test optiques (116) ;
c) la capture, par au moins un dispositif mobile (112) ayant au moins une caméra (114), d'un ensemble d'entraînement d'images, l'ensemble d'entraînement d'images comprenant des images d'au moins une partie d'une ou plusieurs des régions de test de réactifs (120) de l'ensemble d'entraînement de bandelettes de test optiques (116) sur lesquelles est appliqué l'échantillon de fluide corporel (128),
dans lequel au moins deux des bandelettes de test optiques (118) sont non corrompues (122) et dans lequel au moins deux des bandelettes de test optiques sont corrompues (124) ;
d) détermination d'un ensemble d'entraînement de valeurs de formation de couleur à partir des images de l'ensemble d'entraînement d'images, comprenant des valeurs de formation de couleur d'au moins deux canaux de couleur pour la formation de couleur de la région de test de réactifs (120) de chacune des bandelettes de test optiques (118) de l'ensemble d'entraînement de bandelettes de test optiques (116) ; et
e) la dérivation de l'intervalle d'attente de couleur (132) pour les au moins deux canaux de couleur à partir de l'ensemble d'entraînement de valeurs de formation de couleur, l'intervalle d'attente de couleur (132) définissant un intervalle attendu de valeurs de formation de couleur pour les bandelettes de test optiques non corrompues (122).

2. Procédé de détermination selon la revendication précédente, dans lequel la bandelette de test optique corrompue (124) est corrompue par l'un ou les deux parmi une application précédent d'un échantillon de fluide et une exposition précédente à au moins un environnement corrupteur pendant plus de 10 minutes.

3. Procédé de détermination selon la revendication précédente, dans lequel l'environnement corrupteur est un environnement choisi dans le groupe constitué par : un environnement humide et un environnement lumineux.

4. Procédé de détermination selon l'une quelconque des revendications précédentes, dans lequel l'étape d) comprend en outre le marquage des valeurs de formation de couleur de l'ensemble d'entraînement de valeurs de formation de couleur avec des informations indiquant si la bandelette de test optique (118) respective de l'ensemble d'entraînement de bandelettes de test optiques (116) a été corrompue ou non corrompue.

5. Procédé de détermination selon l'une quelconque des revendications précédentes, dans lequel à l'étape e) l'intervalle d'attente de couleur (132) comprend au moins 80 % des valeurs de formation de couleur pour les bandelettes de test optiques non corrompues (122) de l'ensemble d'entraînement de bandelettes de test optiques (116).

6. Procédé de détermination selon l'une quelconque des revendications précédentes, dans lequel la dérivation à l'étape e) comprend la détermination d'une enveloppe comprenant au moins 80 % des valeurs de formation de couleur pour les bandelettes de test optiques non corrompues (122) de l'ensemble d'entraînement de bandelettes de test optiques (116) et en outre l'expansion de l'enveloppe par un facteur de sécurité prédéterminé.

7. Procédé de détermination selon l'une quelconque des revendications précédentes, dans lequel l'étape e) comprend l'utilisation d'au moins un algorithme d'apprentissage machine.

8. Procédé de mesure permettant de réaliser une mesure analytique basée sur une réaction de formation de couleur en utilisant un dispositif mobile (112) ayant une caméra (114) et un processeur (130), le procédé comprenant :
i) la fourniture d'au moins une bandelette de test optique (118) ayant au moins une région de test de réactifs (120) ;
ii) l'application d'un échantillon de fluide corporel (128) sur la région de test de réactifs (120) de la bandelette de test optique (118) ;
iii) la capture, en utilisant la caméra (114), d'au moins une image d'au moins une partie de la région de test de réactifs (120) sur laquelle est appliqué le fluide corporel ;
iv) la détermination d'une valeur de formation de couleur d'au moins deux canaux de couleur pour une formation de couleur de la région de test de réactifs (120) en utilisant l'image ;
v) la comparaison, pour les au moins deux canaux de couleur, de la valeur de formation de couleur avec un intervalle d'attente de couleur (132) bidimensionnel ou tridimensionnel, ayant une forme et/ou un contour arbitraire, déterminé en réalisant le procédé de détermination selon l'une quelconque des revendications précédentes ;
vi) si la valeur de formation de couleur est à l'extérieur de l'intervalle d'attente de couleur (132), la considération que la valeur de formation de couleur n'est pas plausible et l'interruption du procédé de mesure ; et
vii) si la valeur de formation de couleur est à l'intérieur de l'intervalle d'attente de couleur (132), la considération que la valeur de formation de couleur est plausible et la détermination d'une concentration d'analyte dans l'échantillon de fluide corporel en utilisant la valeur de formation de couleur.

9. Procédé de mesure selon la revendication précédente, dans lequel le procédé comprend en outre l'étape viii)de capture, en utilisant la caméra (114), d'au moins une image d'au moins une partie de la région de test de réactifs (120) sur laquelle n'est pas appliqué le fluide corporel, dans lequel l'étape viii) est réalisée avant l'étape ii).

10. Procédé de mesure selon l'une quelconque des deux revendications précédentes, dans lequel le procédé comprend en outre l'étape ix) de fixation de la bandelette de test optique (118) à une carte de référence de couleurs comprenant une pluralité de champs de référence de couleurs ayant des valeurs de couleurs de référence connues, dans lequel l'étape ix) est réalisée avant l'étape iii), et dans lequel l'image capturée à l'étape iii) révèle en outre au moins une partie de la carte de référence de couleurs.

11. Système de détermination (110) permettant de déterminer un intervalle d'attente de couleur (132) bidimensionnel ou tridimensionnel, ayant une forme et/ou un contour arbitraire, pour évaluer la plausibilité d'une valeur de formation de couleur obtenue dans une mesure analytique basée sur une réaction de formation de couleur, comprenant :
A) au moins un dispositif mobile (112) ayant au moins une caméra (114) ;
B) un ensemble d'entraînement de bandelettes de test optiques (116), chaque bandelette de test optique (118) ayant une région de test de réactifs (120), dans lequel au moins deux des bandelettes de test optiques sont non corrompues (122) et dans lequel au moins deux des bandelettes de test optiques sont corrompues (124) ;
C) un ensemble d'entraînement d'échantillons de fluides corporels (126) comprenant une pluralité d'échantillons de fluides corporels (128) ; et
D) au moins un processeur (130), le processeur (130) étant configuré pour
- récupérer un ensemble d'entraînement d'images, l'ensemble d'entraînement d'images comprenant des images, capturées avec la caméra (114), d'au moins une partie de chacune des régions de test de réactifs (120) de l'ensemble d'entraînement de bandelettes de test optiques (116) sur lesquelles est appliqué l'échantillon de fluide corporel (128) ;
- déterminer un ensemble d'entraînement de valeurs de formation de couleur à partir des images de l'ensemble d'entraînement d'images, comprenant des valeurs de formation de couleur d'au moins deux canaux de couleur pour la formation de couleur de la région de test de réactifs (120) de chacune des bandelettes de test optiques (118) de l'ensemble d'entraînement de bandelettes de test optiques (116) ; et
- dériver l'intervalle d'attente de couleur (132) pour les au moins deux canaux de couleur à partir de l'ensemble d'entraînement de valeurs de formation de couleur, l'intervalle d'attente de couleur (132) définissant un intervalle attendu de valeurs de formation de couleur pour les bandelettes de test optiques non corrompues (122).

12. Système de détermination (110) selon la revendication précédente, dans lequel le système de détermination (110) comprend en outre :
E) au moins une carte de référence de couleurs configurée pour avoir la bandelette de test optique (118) fixée de manière amovible à celle-ci, la carte de référence de couleurs comprenant une pluralité de champs de référence de couleurs ayant des valeurs de couleurs de référence connues, dans lequel les images de l'ensemble d'entraînement d'images révèlent en outre au moins une partie de la carte de référence de couleurs.

13. Système de détermination (110) selon l'une quelconque des deux revendications précédentes, dans lequel le système de détermination (110) est configuré pour réaliser au moins les étapes d) et e) du procédé de détermination selon l'une quelconque des revendications précédentes faisant référence à un procédé de détermination.

14. Dispositif mobile (112) ayant au moins une caméra (114) et au moins un processeur (130), le dispositif mobile (112) étant configuré pour réaliser au moins les étapes iv) à vii) du procédé de mesure selon l'une quelconque des revendications précédentes faisant référence à un procédé de mesure.

15. Kit (134) permettant de déterminer la concentration d'au moins un analyte dans un échantillon d'un fluide corporel (128), le kit (134) comprenant le dispositif mobile (112) selon la revendication précédente, le kit (134) comprenant en outre au moins une bandelette de test optique (118) ayant au moins une région de test de réactifs (120).
